Europäisches Patentamt

European Patent Office

Office européen des brevets

(19)

(11) Veröffentlichungsnummer: **0 504 103 B1**

(12) **EUROPÄISCHE PATENTSCHRIFT**

(45) Veröffentlichungstag der Patentschrift: **26.04.95**

(51) Int. Cl.6: **A61B 17/56**, A61F 2/28, A61C 8/00

(21) Anmeldenummer: **92810143.5**

(22) Anmeldetag: **27.02.92**

(54) **Hilfsmittel zum Befestigen und Halten einer Abdeckung an einem Kieferknochen.**

(30) Priorität: **11.03.91 CH 720/91**

(43) Veröffentlichungstag der Anmeldung:
**16.09.92 Patentblatt  92/38**

(45) Bekanntmachung des Hinweises auf die
Patenterteilung:
**26.04.95 Patentblatt  95/17**

(84) Benannte Vertragsstaaten:
**AT BE CH DE DK ES FR GB GR IT LI LU MC NL PT SE**

(56) Entgegenhaltungen:
EP-A- 0 282 789          WO-A-88/01517
WO-A-88/03417          WO-A-90/07308
DE-A- 3 038 319          GB-A- 2 010 095
US-A- 4 787 906

(73) Patentinhaber: **INSTITUT STRAUMANN AG**

**CH-4437 Waldenburg (CH)**

(72) Erfinder: **Buser, Daniel, Dr.**
**Thunerstrasse 12**
**CH-4074 Muri bei Bern (CH)**
Erfinder: **Sutter, Franz, Dr.**
**Bennwilerstrasse 42**
**CH-4435 Niederdorf (CH)**
Erfinder: **Mundwiler, Ulrich**
**Hofmattweg 22**
**CH-4456 Tenniken (CH)**

(74) Vertreter: **Ullrich, Gerhard, Dr.**
**A. Braun, Braun Héritier Eschmann AG**
**Holbeinstrasse 36-38**
**CH-4051 Basel (CH)**

**Beschreibung**

Es ist bekannt, kleinere Unebenheiten eines Kieferknochens mit einer Manschette aus einer geschmeidigen, Gewebe-verträglichen Folie, die z.B. aus Polytetrafluoraethylen oder auch aus einem anderen biokompatiblen Material bestehen kann, zu überziehen, damit sich unter ihr neue Knochensubstanz bilden kann, die sich bis zur aufgespannten Folie erstreckt. Dieses Verfahren wurde bis jetzt nur für die Restaurierung sehr kleiner Untiefen, also Unebenheiten, angewandt, weil man der Meinung war, dass die Knochenbildung nur an solchen Stellen erfolgen werde. Es wurde nun aber ein Hilfsmittel erfunden, mit dem es möglich ist, die Bildung von neuer Knochensubstanz nicht nur bis zu einer Höhe von 1 1/2 bis 2 mm sondern bis zu einer Höhe von bis zu 6 mm zu bewirken. Dieses Hilfsmittel wird gebildet durch einen Stift, der am einen Ende einen Wulst, der mit einer Angriffsfläche zum Angreifen eines Schraubendrehers sowie mit einem Innengewinde und einer darin eingeschraubten Kopfschraube zum Festhalten der Folie versehen ist, aufweist und am andern Ende ein zum Einschrauben in den Kieferknochen bestimmtes Aussengewinde und zwischen dem Wulst und dem Aussengewinde einen zylindrischen, glattwandigen Abschnitt besitzt, dessen Durchmesser grösser ist als der Aussendurchmesser des Aussengewindes. Nachfolgend werden anhand der beiliegenden Zeichnung zwei Ausführungsbeispiele der Erfindung und deren Verwendung beschrieben. In der Zeichnung zeigt

die Fig. 1 einen erfindungsgemässen Stift,

die Fig. 2 die zugehörige Kopfschraube, beides teilweise in Seitenansicht und teilweise im Schnitt,

die Fig. 3 eine Stirnansicht des Stiftes,

die Fig. 4 eine der Fig. 1 entsprechende Darstellung eines zweiten Ausführungsbeispiels, und

die Fig. 5 einen Schnitt durch einen Kieferknochen mit eingesetzten Stiften.

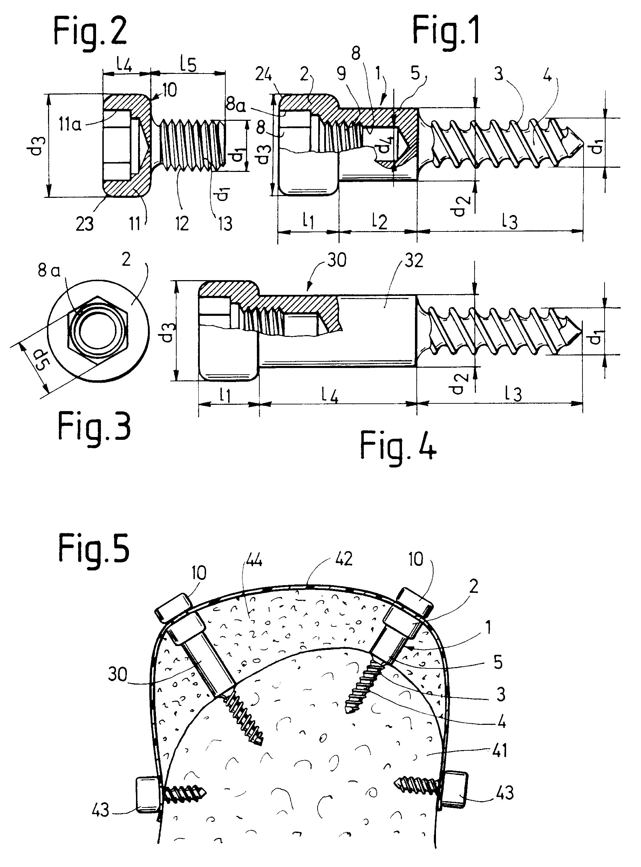

Der in den Fig. 1 und 3 dargestellte, als Ganzes mit 1 bezeichnete Stift weist an dem in der Fig. 1 linken Ende einen Wulst 2 auf. Am gegenüberliegenden, also dem in der Figur rechten Ende ist ein mit einem zum Einschrauben in einen Kieferknochen bestimmten Aussengewinde 3 versehener Abschnitt 4 vorhanden, wobei das Gewinde 3 ein besonders zum Einschrauben in den Knochen geeignetes an sich bekanntes Profil aufweist. Zwischen diesem mit dem Aussengewinde 3 versehenen Abschnitt 4 und dem Wulst 2 befindet sich ein zylindrischer, glattwandiger Abschnitt 5, dessen Durchmesser $d_2$ mindestens gleich gross, vorzugsweise aber etwas grösser ist als der Aussendurchmesser $d_1$ des Aussengewindes 3. Von der linken Seite erstreckt sich ein Sackloch in den Stift 1. Dieses Sackloch 8 weist in seinem äussersten Abschnitt eine Innensechskantfläche 8a auf, die zum Einstecken eines sechskantigen Schraubendrehers dient und deren Abmessungen daher mit der entsprechenden Norm übereinstimmen. Das Sackloch 8 weist des weitern ein Innengewinde 9 auf, das sich so weit nach innen erstreckt, dass die in der Fig. 2 dargestellte, als Ganzes mit 10 bezeichnete Schraube so weit eingeschraubt werden kann, bis ihr Schraubenkopf 11 am Wulst 2 ansteht, wobei der Schraubenkopf 11 zweckmässigerweise so ausgebildet und dimensioniert ist, dass er mit dem Wulst 2 übereinstimmt, also den gleichen Durchmesser $d_3$ und die gleich ausgebildeten Angriffsflächen 11a für den Schraubendreher aufweist wie der Wulst 2. Der Durchmesser des mit dem Gewinde 12 versehenen Abschnitts 13 der Schraube 10 ist so bemessen, dass die Wandung des Sackloches 8 im Bereich des dem Aussengewinde 12 entsprechenden Innengewinde 9 eine hinreichende Wandstärke hat. Als zweckmässig haben sich die folgenden Abmessungen erwiesen:

2

$d_1$ Durchmesser des Aussengewindes 3 am Stift 1          1,2  mm

$d_2$ Durchmesser des zylindrischen Abschnitts 5            1,8  mm

$d_3$ Durchmesser des Wulstes 2 des Stiftes 1          ⎫
                                                       ⎬    2,5  mm
Durchmesser des Kopfes 11 der Schraube 10              ⎭

$d_1$ Durchmesser des Gewindes 12 der Schraube 10          1,2  mm

$d_4$ Durchmesser des gewindefreien Teils des

Sackloches 8                                               1    mm

$d_5$ Flächenabstand im Sechs-Kantloch                     0,87 mm

$L_1$ Axiale Länge des Wulstes 2                           1,5  mm

$L_2$ Axiale Länge des Abschnitts 5                        2    mm

$L_3$ Axiale Länge des Abschnitts 4                        4    mm

$L_4$ Axiale Länge des Schraubenkopfes 11                  1,2  mm

$L_5$ Axiale Länge der ganzen Schraube 10                  3    mm

Die Fig. 4 zeigt eine zweite Ausführungsform eines erfindungsgemässen Stiftes. Dieser als Ganzes mit 30 bezeichnete Stift stimmt mit dem vorstehend beschriebenen Stift 1 in bezug auf alle Angaben und Grössenverhältnisse überein, ausgenommen in Bezug auf die axiale Länge $L_4$ des hier mit 32 bezeichneten glattwandigen, zylindrischen Abschnitts. Dieser ist hier nämlich doppelt so lang wie der entsprechende Abschnitt 5 im ersten Ausführungsbeispiel. Natürlich wäre es ohne weiteres möglich, je nach Bedarf die Stifte mit einem etwas längern oder kürzern Abschnitt zu versehen.

Des weitern wäre es bei allen Ausführungsbeispielen ohne weiteres möglich, anstelle der Innensechskantfläche zum Angreifen des Schraubendrehers eine Innendreikant-, Vierkantoder Fünfkant-Fläche vorzusehen oder im Rand 24 des Stiftes und im entsprechenden Rand 23 der Schraube 10 zwei radial verlaufende Schlitze anzubringen, die sich zum Angreifen eines gewöhnlichen Schraubendrehers eignen.

Die Fig. 5 zeigt nun, wie das vorstehend beschriebene Hilfsmittel verwendet werden kann:

In den mit 41 bezeichneten Kieferknochen werden zwei erfindungsgemässe Stifte, und zwar ein Stift 1 und ein Stift 30, soweit eingeschraubt, dass sich der ganze Gewindeabschnitt 4 jedes Stiftes innerhalb des Knochens befindet. Dann wird eine Gewebe-verträgliche Abdeckfolie 42, zweckmässigerweise aus Polytetrafluoraethylen, über diese Stifte gelegt und an ihrem Rand mit Schrauben 43, von denen in der Zeichnung nur zwei dargestellt sind, am Kieferknochen befestigt. Nachher werden zur Sicherung der Folie die Schrauben 10 in die Stifte 1 und 30 eingeschraubt. Wie die Erfahrung gezeigt hat, wird sich nun zwischen dem Knochen 41 und der Folie 42 Knochenmasse 44 aufbauen und zwar soweit, bis der ganze Raum ausgefüllt ist. Dann kann man die Folie, die Schrauben und die Stifte wieder entfernen und somit der Schleimhaut die Möglichkeit geben, den Knochen wieder zu überwachsen.

**Patentansprüche**

1. Zum Befestigen und Halten einer das Zuwachsen einer in einem Kieferknochen vorhandenen Vertiefung fördernden Abdeckung aus einer Gewebe-verträglichen Folie dienendes Hilfsmittel, das als Stift ausgebildet ist und an einem Ende einen Wulst mit einer aufgeschraubten Kopfschraube aufweist, dadurch gekennzeichnet, dass der Wulst (2) mit einer Angriffsfläche (8a) zum Angreifen eines Schraubendrehers versehen ist, dass am andern Ende des Stiftes ein zum Einschrauben in den Kieferknochen (41) bestimmtes Aussengewinde (3) vorhanden ist und, dass zwischen dem Wulst (2) und dem Aussengewinde (3) ein zylindrischer, glattwandiger Abschnitt (5) ausgebildet ist, dessen Durchmesser ($d_2$) grösser ist als der Aussendurchmesser ($d_1$) des Aussengewindes (3).

2. Hilfsmittel nach Anspruch 1, dadurch gekennzeichnet, dass die Angriffsfläche (8a) zum Ansetzen eines Schraubendrehers eine Innenvierkant- oder Innensechskantfläche ist.

**3.** Hilfsmittel nach einem der Ansprüche 1 oder 2, dadurch gekennzeichnet, dass die Kopfschraube (10) eine Angriffsfläche (11a) zum Ansetzen eines Schraubendrehers hat, die gleich ausgebildet ist wie die zum Angreifen eines Schraubendrehers am Wulst (2) angebrachte Angriffsfläche (8a).

**4.** Hilfsmittel nach einem der Ansprüche 1 bis 3, dadurch gekennzeichnet, dass der zylindrische Abschnitt (5) eine axiale Länge ($L_4$) von mindestens 2 mm aufweist.

**5.** Hilfsmittel nach einem der Ansprüche 1 bis 4, dadurch gekennzeichnet, dass die Länge ($L_3$) des Abschnitts (4) mit dem Aussengewinde (3) 3 - 5 mm beträgt.

**6.** Hilfsmittel nach einem der Ansprüche 1 bis 5, dadurch gekennzeichnet, dass das Aussengewinde (3) einen Aussendurchmesser ($d_1$) von 1,2 mm besitzt.

**7.** Hilfsmittel nach Anspruch 6, dadurch gekennzeichnet, dass der zylindrische Abschnitt (5) einen Durchmesser ($d_2$) von 1,8 mm aufweist.

**8.** Hilfsmittel nach Anspruch 7, dadurch gekennzeichnet, dass der Wulst (2) einen Durchmesser ($d_3$) von 2,5 mm aufweist.

## Claims

**1.** Aid which serves for the fastening and retention of a covering of a tissue-compatible foil required for the growing out of a depression in a jawbone and which is constructed as a pin and has at one end a bead with a screwed-on cap screw, characterised thereby that the bead (2) is provided with an engagement surface (8a) for the engagement of a screw turning tool, that an external thread (3) intended for screwing into the jawbone (41) is present at the other end of the pin, and that formed between the bead (2) and the external thread (3) is a cylindrical, smooth-walled portion (5), the outer diameter ($d_2$) of which is larger than the outer diameter ($d_1$) of the outer thread (3).

**2.** Aid according to claim 1, characterised thereby that the engagement surface (8a) is an internal square or internal hexagonal surface for the application of a screw turning tool.

**3.** Aid according to one of claims 1 or 2, characterised thereby that the cap screw (10) has an engagement surface (11a) for the application of a screw turning tool, which is constructed to be the same as the engagement surface (8a) formed at the bead (2) for the engagement of a screw turning tool.

**4.** Aid according to one of claims 1 to 3, characterised thereby that the cylindrical portion (5) has an axial length ($L_4$) of at least 2 mm.

**5.** Aid according to one of claims 1 to 4, characterised thereby that the length ($L_3$) of the portion (4) with the external thread (3) amounts to 3 to 5 mm.

**6.** Aid according to one of claims 1 to 5, characterised thereby that the external thread (3) has an outer diameter ($d_1$) of 1.2mm.

**7.** Aid according to claim 6, characterised thereby that the cylindrical portion (5) has a diameter ($d_2$) of 1.8mm.

**8.** Aid according to claim 7, characterised thereby that the bead (2) has a diameter ($d_3$) of 2.5mm.

## Revendications

**1.** Accessoire servant à fixer et maintenir une couverture en feuille histocompatible favorisant le comblement d'une dépression présente dans un maxillaire, lequel est constitué par une broche et présente à une extrémité un bourrelet comportant une vis à tête vissée, caractérisé en ce que le bourrelet (2) est doté d'un plat d'entraînement (8a) permettant l'entraînement par un tournevis, que l'autre extrémité de la broche présente un filet mâle (3) destiné à être vissé dans le maxillaire (41) et qu'entre le bourrelet (2) et le filet mâle (3) est formé un tronçon cylindrique à paroi lisse (5) dont le diamètre (d$_2$) est supérieur au diamètre externe (d$_1$) du filet mâle (3).

2. Accessoire selon la revendication 1, caractérisé en ce que le plat d'entraînement (8a) destiné à la mise en place d'un tournevis est un quatre pans creux ou un six pans creux.

3. Accessoire selon une des revendications 1 et 2, caractérisé en ce que la tête de vis (10) possède un plat d'entraînement (11a) permettant la mise en place d'un tournevis, lequel a une configuration identique à celle du plat d'entraînement (8a) ménagé sur le bourrelet (2) en vue de l'entraînement par un tournevis.

4. Accessoire selon une des revendications 1 à 3, caractérisé en ce que le tronçon cylindrique (5) présente une longueur axiale (L$_4$) d'au moins 2 mm.

5. Accessoire selon une des revendications 1 à 4, caractérisé en ce que la longueur (L$_3$) du tronçon (4), filet mâle (3) compris, est de 3 à 5 mm.

6. Accessoire selon une des revendications 1 à 5, caractérisé en ce que le filet mâle (3) possède un diamètre externe (d$_1$) de 1,2 mm.

7. Accessoire selon la revendication 6, caractérisé en ce que le tronçon cylindrique (5) présente un diamètre (d$_2$) de 1,8 mm.

8. Accessoire selon la revendication 7, caractérisé en ce que le bourrelet (2) présente un diamètre (d$_3$) de 2,5 mm.

Fig.2

Fig.1

Fig.3

Fig.4

Fig.5